**Europäisches Patentamt**

**European Patent Office**

**Office européen des brevets**

(19)

(11) Veröffentlichungsnummer: **0 133 308**
**B1**

(12) **EUROPÄISCHE PATENTSCHRIFT**

(45) Veröffentlichungstag der Patentschrift:
**17.02.88**

(21) Anmeldenummer: **84108919.6**

(22) Anmeldetag: **27.07.84**

(51) Int. Cl.⁴: **C 07 K 15/14, A 61 K 37/02**

(54) **Appetitregulierender Wirkstoff und Verfahren zur Herstellung desselben.**

(30) Priorität: **29.07.83 HU 271883**

(43) Veröffentlichungstag der Anmeldung:
**20.02.85 Patentblatt 85/8**

(45) Bekanntmachung des Hinweises auf die Patenterteilung:
**17.02.88 Patentblatt 88/7**

(84) Benannte Vertragsstaaten:
**AT BE CH DE FR GB IT LI NL SE**

(56) Entgegenhaltungen:
**EP - A - 0 075 925**
**US - A - 4 294 825**

**JOURNAL OF CHROMATOGRAPHY, Band 317, 28. Dezember 1984, Seiten 165-172, Elsevier Science Publishers B.V., Amsterdam, NL; & EIGHTH INTERNATIONAL SYMPOSIUM ON COLUMN LIQUID CHROMATOGRAPHY, New York, NY, US, 20.-25. Mai 1984, PART II; J. NAGY et al.: "Molecular weight measurement of human satietin"**
**CHEMICAL ABSTRACTS, Band 101, Nr. 9, 27. August 1984, Seite 70, Nr. 66118e, Columbus, Ohio, US; J. KNOLL: "Satietin; a 50,000 dalton glycoprotein in human serum with potent, long-lasting and selective anorectic activity" & J. NEURAL TRANSM. 1984, 59(3), 163-94**

(73) Patentinhaber: **RICHTER GEDEON VEGYESZETI GYAR R.T., Gyömröi ut 19-21, H-1103 Budapest X (HU)**

(72) Erfinder: **Knoll, Jozsef, Dr., Jászai Mari tér 4/b, Budapest XIII (HU)**
Erfinder: **Nagy, János, Dr. Dipl.-Chem., Jokai u. 2, Szentendre (HU)**
Erfinder: **Kalász, Huba, Dipl-Chem. Dr., Hevesi Gy. u. 95, Budapest XVI (HU)**
Erfinder: **Knoll, Berta, Dr., Madách tér 2-6, Budapest VII (HU)**

(74) Vertreter: **Hansen, Bernd, Dr.rer.nat. et al, Hoffmann, Eitle & Partner Patentanwälte Arabellastrasse 4, D-8000 München 81 (DE)**

(56) Entgegenhaltungen: (Fortsetzung)
**CHEMICAL ABSTRACTS, Band 98, Nr. 15, 11. April 1983, Seite 88, Nr. 119817k, Columbus, Ohio, US; NAGY, JANOS et al.: "An improved method for the preparation of highly purified satietin samples from human serum" & POL. J. PHARMACOL. PHARM. 1982, 34(1-3), 47-52**
**CHEMICAL ABSTRACTS, Band 91, Nr. 25, 17. Dezember 1979, Seite 393, Nr. 208025y, Columbus, Ohio, US; J. KNOLL: "Satietin: a highly potent anorexogenic substance in human serum" & PHYSIOL. BEHAV. 1979, 23(3), 497-502**

## Beschreibung

Die Erfindung betrifft einen appetitregulierenden Wirkstoff, der spezifisch auf das Appetitzentrum wirkt, sowie ein Verfahren zur Herstellung desselben.

In der ungarischen Patentschrift Nr. 178 703 ist ein Verfahren beschrieben, mit dessen Hilfe ein durch einen bisher nicht bekannten Wirkungsmechanismus wirkender, selektiv appetitzügelnder Stoff aus menschlichem Blutplasma hergestellt werden kann. Dieser Stoff wurde als Satietin bezeichnet; es handelte sich um ein partiell gereinigtes Produkt, das jedoch in der Wirkung die bisher bekannten Substanzen ähnlicher Wirkungsrichtung schon weit übertraf.

Die Reinigung des Produktes erfolgte mittels Ultrafiltration des Plasmas und einer zweistufigen Gelchromatographie an den Gelen Sephadex G-15 und Bio-Gel P-2.

Es gelang, die Peptidnatur des Stoffes nachzuweisen und die Mengenverhältnisse der in grösseren Mengen enthaltenen Aminosäuren zu bestimmen. Ausser den Aminosäuren wurden nach Hydrolyse des Produktes auch Zuckerkomponenten gefunden, und schon damals wurde vermutet, dass es sich bei dem spezifisch auf das Appetitzentrum wirkenden Produkt um ein Glycoprotein handelt.

Bei der Weiterentwicklung dieser Erfindung wurde gefunden, dass mittels eines weiteren Verfahrens die wirksame Fraktion weiter zerlegt werden kann. (Dieses weiterentwickelte Verfahren ist Gegenstand einer eigenen früheren Anmeldung, der ungarischen Patentanmeldung Nr. 2783/81, die am 3. März 1982 modifiziert wurde.) Gemäss dem dort beschriebenen Verfahren kann ein Produkt hergestellt werden, das drei- bis viermal wirksamer ist und auch in der chemischen Zusammensetzung von dem mit dem ersten Verfahren hergestellten Produkt wesentlich abweicht. Dieses wirksamere Produkt ist bereits als chemisch reine, einheitliche Substanz zu betrachten, deren physikalische Parameter und chemische Zusammensetzung bestimmt wurden.

Im Sinne dieser früheren Erfindung wurde das menschliche Plasma nach der Ultrafiltration einem wesentlichen Arbeitsgang unterzogen: das Plasmafiltrat wurde mit Trichloressigsäure behandelt, wodurch die noch im Filtrat befindlichen Serumproteine (Albumin usw.), die in gewisser Menge die Amicon-Membran zu durchdringen vermögen, entfernt werden können. Die mit Trichloressigsäure ausgefällten Serumproteine wurden durch Zentrifugieren abgesetzt, und der die biologisch aktiven Satietinfraktionen enthaltende klare Überstand wurde an einer Gelsäule weiter gereinigt. Im ersten Schritt wurde an der Sephadex G-15 Säule mit Ammoniumacetat-Puffer eluiert, und beim Ausschlussvolumen wurden diejenigen Fraktionen gesammelt, die die Aktivität enthielten. Diese Fraktionen wurden nach entsprechender Einengung an einer Säule aus Bio-Gel P-2 entsalzt. Die salzfreien aktiven Fraktionen konnten schon als hochreiner Stoff zu biologischen Versuchen – einschliesslich sämtliche Experimente zur Nahrungsaufnahme – verwendet werden.

In dieser Phase waren nur noch die die Satietinaktivität nicht beeinflussenden Peptide und Glycopeptide geringeren Molekulargewichtes enthalten; diese wurden dann mittels Elektrophorese und Affinitätschromatographie entfernt. Mit der Affinitätschromatographie wurde die Substanz an einer Säule aus Con-A Sepharose fraktioniert und an einer Säule aus Bio-Gel P-2 erneut entsalzt; der reine Stoff wurde lyophilisiert. Das erhaltene Produkt erwies sich als ein Glycoprotein mit geringem Peptidgehalt (5–25%) und hohem Kohlenhydratanteil (60–90%). Das Molekulargewicht des Wirkstoffes fiel in den Bereich zwischen 50 000 und 70 000, sein isoelektrischer Punkt lag im Neutralbereich (pI: 7,0–7,1).

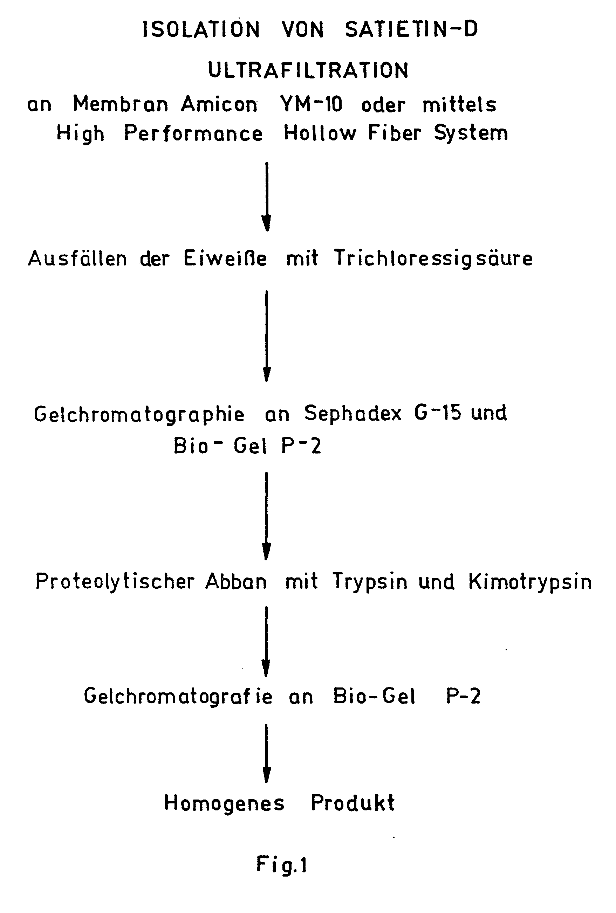

Neuere Untersuchungen und die Weiterentwicklung der Erfindung führten zu der Erkenntnis, dass das beschriebene Verfahren stark vereinfacht werden kann und durch Einschaltung eines speziellen Schrittes ein in den physikalischen Eigenschaften abweichendes, d.h. neues Produkt isolierbar ist, das die Nahrungsaufnahme spezifisch hemmt. Diese Substanz erhielt die Bezeichnung Satietin-D. Es wird auf die in Fig. 1 veranschaulichte Weise folgendermassen isoliert.

Von Menschen und/oder Säugetieren stammendes Blutserum wird an einem bis zum Bereich von 50 000 Dalton Molekulargewicht durchlässigen Membranfilter filtriert. Diese Membranfiltration bzw. Ultrafiltration kann an Säulen (sog. «hollow fiber Technik») vorgenommen werden. Dann wird das Filtrat eingedampft und der unlösliche Teil – zweckmässig durch Zentrifugieren oder Filtrieren – abgetrennt. Zu der flüssigen Phase wird bei einer Temperatur von 0–10°C Trichloressigsäure bis zu einer Konzentration von 5–25 G/V-%, vorzugsweise 9–11 G/V-%, gegeben (G/V-% = Gewicht/Volumen-%). Die ausgefällten Proteine werden – zweckmässig durch Zentrifugieren oder Filtrieren – entfernt, und die erhaltene klare Lösung wird an einem Gel mit einem Ausschlussvolumen von unter 4000 Dalton chromatographiert. Eluiert wird mit Pufferlösung pH 6,0–7,0, die biologisch aktiven Fraktionen werden – zweckmässig durch Lyophilisieren oder Eindampfen im Vakuum – konzentriert und dann an einem Gel mit einem Ausschlussvolumen von unter 3000–4000 Dalton rechromatographiert, mit destilliertem Wasser eluiert (entsalzt) und das erhaltene Rohprodukt in lyophilisierter Form der weiteren Reinigung bzw. Behandlung unterzogen.

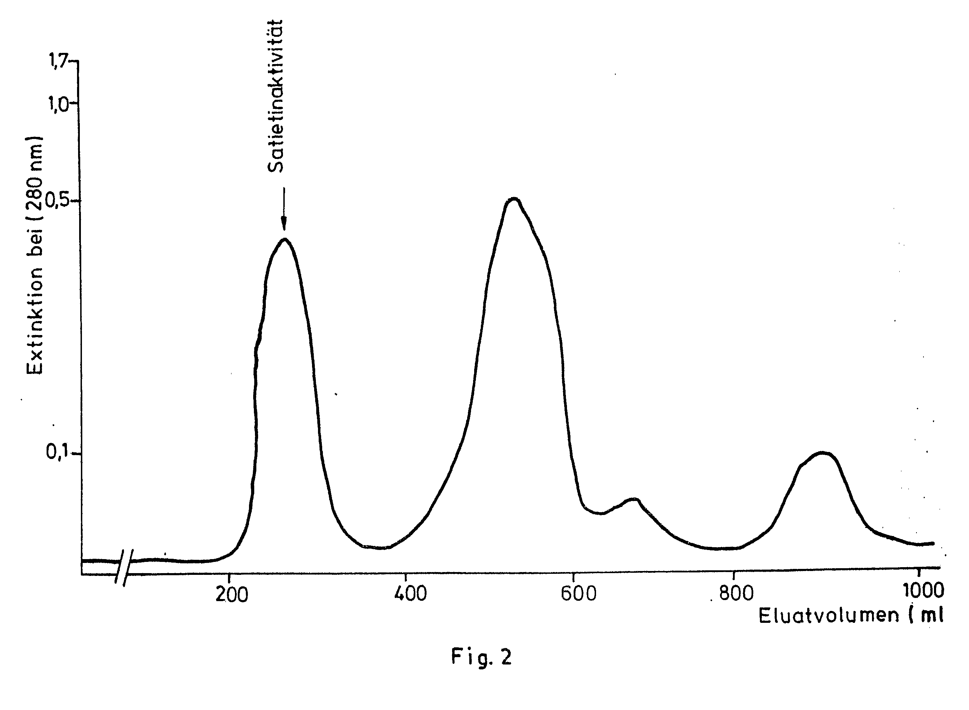

Im Sinne der Erfindung wird das lyophilisierte Rohprodukt in einem Puffer von pH 8,1–8,2, zweckmässig in 0,1 molarer TRIS-Hydrochloridlösung (2-Amino-2-hydroxymethyl-1,3-propandiolhydrochlorid) gelöst und, auf das Gesamtgewicht bezogen, mit 0,01–0,2 Teilen, vorzugsweise einem Zehntel des Gewichtes, an Trypsin und der gleichen Menge Chymotrypsin versetzt. Das Reaktionsgemisch wird dann bei 37–38°C 20–30 Stunden, vorzugsweise 24 Stunden lang, zweckmässig unter gelegentlichem Rühren oder kontinuierlichem Schütteln, dem enzymatischen Abbau unterzogen. Nach Ablauf der ersten 5 Stunden wird noch einmal die halbe Menge Trypsin und Chymotrypsin zugesetzt und der enzymatische Abbau fortgesetzt. Dann wird aus dem Reaktionsgemisch durch Behandeln mit kalter Trichloressigsäure und Chromatographie an einem Gel mit einem Ausschlussvolumen von unter 3000-4000 Dalton der reine Wirkstoff in salzfreier Form isoliert. Hierzu wird auf Fig. 2, die das entsprechende Extinktionsdiagramm wiedergibt, hingewiesen.

Im folgenden wird das Verfahren ausführlicher beschrieben. Der Reinigungsprozess wird mit der Ultrafiltration begonnen, wozu ein Amicon «hollow fiber» Gerät – zweckmässig unter Druck bzw. mit einer geeigneten Pumpe – eine bis zu 50 000 Dalton separierende Säule verwendet wird. Das erhaltene Ultrafiltrat wird durch Eindampfen im Vakuum konzentriert und der unlösliche Teil mittels einer präparativen Zentrifuge abgetrennt oder durch eine Zeiss-Platte mittels Vakuum abfiltriert. Das niederschlagfreie Gemisch wird auf 0-10°C gekühlt und mit so viel 55%iger Trichloressigsäure versetzt, dass deren Konzentration im Gemisch 5-25 G/V-%, vorzugsweise 9-11 G/V-%, beträgt. Die behandelte Lösung wird bei 0-5°C etwa eine Stunde stehen gelassen, dann wird der Niederschlag bzw. die Trübung durch Ultrazentrifugieren oder durch Vakuumfiltration durch ein Zeiss-Filter abgetrennt. Die erhaltene Lösung ist klar und von lebhaft gelber Farbe. Wie bereits aus den oben zitierten Publikationen bekannt, wird durch die Trichloressigsäure das noch vorhandene Eiweiss, so auch das Albumin, ausgefällt, während der Wirkstoff, das Satietin, in Lösung bleibt.

Für den folgenden Reinigungsschritt wird präparative Gelchromatographie an einer Gelsäule mit einem Ausschlussvolumen von unter 4000 Dalton Molekulargewicht eingesetzt. Solche Gele sind zum Beispiel Sephadex G-15, G-10 und G-25. Zum Eluieren wird vorzugsweise 0,1 molare Ammoniumacetatlösung verwendet, weil das Ammoniumacetat infolge seiner Flüchtigkeit beim Lyophilisieren verhältnismässig leicht entfernbar ist. Es können auch 0,9%ige (physiologische) Kochsalzlösung oder unterschiedliche Phosphatpuffer verwendet werden. Die aktiven Fraktionen erscheinen beim Ausschlussvolumen der Gelsäule (sie werden bei dem etwa einem Drittel des Säulenvolumens entsprechenden Elutionsmittelvolumen aus dem Gel verdrängt). Diese Fraktionen werden vereinigt und durch Lyophilisieren konzentriert.

Im folgenden Reinigungsschritt wird ebenfalls Gelchromatographie, hier an einem Gel mit einem Ausschlussvolumen von unter 3000 Dalton, zweckmässig Bio-Gel P-2, eingesetzt, wobei das Gel mit destilliertem Wasser eluiert wird. In diesem Schritt werden die Salze und andere eventuell vorhandene niedermolekulare Verunreinigungen entfernt. Die beim Ausschlussvolumen austretenden Fraktionen mit Satietinaktivität werden vereinigt und dann lyophilisiert. Das erhaltene Produkt ist ein blassgelbes oder weisses Pulver mit dem isoelektrischen Punkt pI = 7,0-7,1. Aus einem Liter menschlichen Blutserum oder Plasma werden auf diese Weise 8-10 mg lyophilisiertes Produkt erhalten, das zur Regulierung des Appetits bereits geeignet ist, da seine Satietinaktivität etwa 25 SE/mg beträgt.

Die Satietinaktivität wird mit einer eigens dafür ausgearbeiteten biologischen Wertmessmethode bestimmt: unter einer Satietineinheit (1 SE) wird diejenige Wirkstoffmenge verstanden, die – 96 Stunden lang ausgehungerten weiblichen CFY-Ratten von 200-240 g Gewicht intracerebroventrikular verabreicht – den am ersten Fütterungstag erfolgenden Verzehr von pelletiertem Standardfutter von 24,04±0,76 g auf 10 g vermindert.

Das erhaltene satietinaktive Rohprodukt kann chemisch noch nicht als reiner Stoff betrachtet werden, weil bei Untersuchung mit elektrophoretischen Methoden, zum Beispiel Gelelektrophorese an Polyacrylamid in Gegenwart von Dodecylsulfat oder bei isoelektrischer Fokussierung, mehrere Eiweissanfärbungen ein positives Ergebnis zeigen.

Zur Herstellung des einheitlichen Wirkstoffes wurde ein verglichen mit der früheren Methode wirksameres und einfacheres Verfahren ausgearbeitet. In diesem Schritt des erfindungsgemässen Verfahrens wird proteolytischer Abbau angewendet, und zwar aus der Überlegung heraus, dass im Zuge der Verdauung die als verunreinigende Komponenten noch enthaltenen Peptide und Eiweisse zersetzt werden, während das Satietin als Glycoprotein gegen proteolytische Enzyme wesentlich widerstandsfähiger ist. Das im vorherigen Schritt des Verfahrens erhaltene, bereits hochreine Produkt wird in einem schwach alkalischen Puffer (pH 8,1-8,2), vorzugsweise in 0,1 molarer TRIS-Hydrochloridlösung, unter Schütteln oder Rühren aufgelöst. Zu der Lösung werden, auf das Gewicht des lyophilisierten Produktes bezogen, 0,01-0,2, vorzugsweise 0,1 Gew.-Teile Trypsin und die gleiche Menge Chymotrypsin gegeben. Das Gemisch wird bei 36-39°C, vorzugsweise 37-38°C, 20-30 Stunden, vorzugsweise 24 Stunden, lang geschüttelt beziehungsweise gerührt. Nach Beginn der enzymatischen Zersetzung wird nach Ablauf von 5 Stunden erneut Trypsin und Chymotrypsin zugesetzt, aber nun nur die Hälfte der zuerst zugesetzten Menge. Nach insgesamt etwa 24 Stunden wird das Gemisch auf 0-10°C gekühlt und mit so viel Trichloressigsäure versetzt, dass deren Konzentration in dem Gemisch 4-6 G/V-%, vorzugsweise 5 G/V-%, beträgt. Dann lässt man das Gemisch bei Temperaturen zwischen –15°C und +5°C 1-24 Stunden stehen. Anschliessend wird der unlösliche Teil zweckmässig durch Ultrazentrifugieren oder Filtration durch ein Zeiss-Filter abgetrennt.

Im folgenden Schritt der Reinigung werden die Trichloressigsäure, die Salze, die verwendeten Puffer und sonstige niedermolekulare Fragmente entfernt. Der Überschuss der Enzyme wurde bereits durch die Trichloressigsäure abgetrennt. Die reine, klare Lösung wird an einem Gel mit einem Ausschlussvolumen von 3000–4000 Dalton Molekulargewicht vorzugsweise mit Wasser eluiert. Das reine, homogene Satietin wird aus dem Gel verdrängt und erscheint in den einem Drittel des Gelvolumens entsprechenden Eluatfraktionen (Fig. 2). Die mit ionenfreiem Wasser erhaltenen, wirkstoffhaltigen Fraktionen werden vereinigt und lyophilisiert. Das Produkt ist ein schneeweisses Pulver, völlig rein und einheitlich. Es wird als Satietin-D bezeichnet, denn es unterscheidet sich in seinen physikalischen und chemischen Eigenschaften von dem bereits früher hergestellten homogenen Satietinprodukt.

Das Molekulargewicht des auf die erfindungsgemässe Weise isolierten einheitlichen und reinen Wirkstoffes Satietin-D wurde mittels Gelelektrophorese (SDS/Natriumdodecylsulfat) zu 40 000–50 000 Dalton bestimmt. Die gleiche Methode brachte auch den Beweis für die Reinheit und Homogenität des Stoffes, da es bei Eiweissanfärbung und Kohlenhydratanfärbung einen einzigen Streifen ergab. Auch das ist ein Beweis für die Glycoproteinnatur des Stoffes. Die isoelektrische Fokussierung des Stoffes in Gegenwart von Ampholin bei pH-Werten von 3–10 bzw. 3–5 ergab, dass der isoelektrische Punkt bei 2,9–3,1 liegt. Dies besagt, dass der aus dem menschlichen Serum isolierte Stoff von den bisher bekannten abweicht und deshalb als neue Substanz betrachtet werden muss.

Bei der chemischen Analyse des Stoffes wurden folgende Werte gefunden:

| | |
|---|---|
| Eiweiss | 20–23% |
| Kohlenhydrate | 56–60% |
| chemisch nicht gebundenes Wasser | 6–10% |

Der Eiweissgehalt wurde mit der Mikrobiuret-Methode bestimmt. Nach Hydrolyse des Produktes wurde eine Aminosäureanalyse vorgenommen, die folgendes Ergebnis zeigte: Asp 2,83%, Thr 1,61%, Ser 1,25%, Glu 3,60%, Pro 1,36%, Gly 0,87%, Ala 1,18%, Cys 0,16%, Val 0,93%, Met 0,17%, Ile 0,76%, Leu 1,25%. Tyr 1,42%, Phe 0,77%, Lys 3,67%, His 0,22%, Arg 0,67%; d.h. Gesamtgehalt an Aminosäuren: 22,71%; Glycosamin: 4,90%.

Die Kohlenhydrate verteilten sich in einer Probe wie folgt: Fukose 4,5%, Mannose 8,05%, Galactose 30,3%, Glucose 14,4%; insgesamt: 56,5%.

Aus den Parametern und Analysewerten ergibt sich eindeutig, dass es sich bei dem erfindungsgemäss durch proteolytischen Abbau erhaltenen Produkt um ein neues, bisher nicht bekanntes Glycoprotein handelt. Aus einem Liter menschlichen Serums können etwa 4–6 mg reines, einheitliches Produkt gewonnen werden, dessen Satietinaktivität 50–100 SE/mg beträgt.

Das erfindungsgemässe Verfahren wird anhand der folgenden Beispiele näher erläutert, ist jedoch nicht auf diese Beispiele beschränkt.

Beispiel

a) 300 ml menschliches Blutserum werden unter ständigem Rühren und 3–4 at Druck durch eine bis zu 30 000 Dalton durchlässige Amicon «hollow fiber» Säule filtriert. Die erhaltenen 2000 ml Ultrafiltrat werden im Vakuum auf 60 ml Volumen eingedampft. Das Niederschlag enthaltende Konzentrat wird mit 9000 g 30 Minuten lang zentrifugiert. Der klare Überstand wird abgetrennt und unter Rühren und Kühlen tropfenweise mit 12 ml 55%iger Trichloressigsäure versetzt. Das Gemisch wird bei 5–10°C eine Stunde lang stehen gelassen und dann zwecks Entfernung der ausgefällten Eiweisse bei etwa 5°C mit 30 000 g so lange zentrifugiert, bis die überstehende Flüssigkeit optisch völlig klar ist. Die Flüssigkeit wird an einer mit Sephadex G-15 gefüllten Säule (5×90 cm) chromatographiert, wobei mit 0,1 molarer Ammoniumacetat-Pufferlösung (pH = 6,6) eluiert wird. Die Fraktionen zwischen 500 und 600 ml werden vereinigt und lyophilisiert. Das Lyophilisat wird in 10 ml destilliertem Wasser gelöst und auf eine Gelsäule (2,5×90 cm, Bio-Gel P-2) aufgebracht. Die Säule wird mit destilliertem Wasser eluiert, die Fraktionen zwischen 130 ml und 180 ml werden vereinigt und lyophilisiert. Auf diese Weise erhält man 30 mg rohes, salzfreies Satietin in Form eines gelblichweissen Pulvers.

b) Aus 100 mg des gemäss a) erhaltenen Rohproduktes wird mit 0,1 molarer TRIS-Hydrochloridlösung (pH = 8,2) bei Raumtemperatur unter Schütteln oder Rühren eine Lösung der Konzentration von 5 mg/ml bereitet. Zu der schwach opalisierenden Lösung werden 10 mg Trypsin und gleichzeitig 10 mg Chymotrypsin gegeben, dann wird das Gemisch gründlich gerührt. Das noch ein wenig Niederschlag enthaltende Gemisch wird auf ein Bad von 37°C Temperatur gestellt und gelegentlich umgerührt beziehungsweise geschüttelt. Nach 5 Stunden gibt man weitere 5 mg Trypsin und 5 mg Chymotrypsin zu und inkubiert das Gemisch dann bei 37°C weitere 19 Stunden. Nach Beendigung des enzymatischen Abbaus wird das Gemisch auf 0–5°C gekühlt und mit 0,1 Vol.-%, d.h. mit 2 ml 55 Gew.-%iger, kalter Trichloressigsäure versetzt. Das Gemisch wird geschüttelt und dann bei 0–5°C eine Stunde lang stehen gelassen. Das etwas Niederschlag enthaltende Gemisch wird dann bei der gleichen Temperatur 25 Minuten lang ultrazentrifugiert. Der klare Überstand wird bei Raumtemperatur auf eine Gelsäule (5.0×45 cm, Bio-Gel P-2) aufgebracht und mit destilliertem Wasser eluiert. Die Fraktionen zwischen 250 und 320 ml werden vereinigt und lyophilisiert. Man erhält 62 mg schneeweisses, salzfreies, reines Satietin-D, dessen biologische Aktivität 50–100 SE/mg beträgt.

Patentansprüche

1. Appetitregulierender Wirkstoff (Satietin D), der spezifisch auf das Appetitzentrum wirkt, und aus Blutserum gewonnen wird, dadurch erhältlich, dass man das menschliche und/oder tierische Blutserum durch ein bis höchstens zum Molekulargewicht 50 000 durchlässiges Ultrafilter filtriert, das Filtrat partiell eindampft und dann bei 0–10°C bis zum Erreichen einer Konzentration von 5–25 G/V-%, vorzugsweise 9–11 G/V-%, mit Trichloressigsäure versetzt, die ausgefällten Proteine entfernt, die erhaltene Lösung an einem Gel mit einem Ausschlussvolumen von unter 4000 chromatographiert, mit einer Lösung eines pH-Wertes von 6,0–7,0 eluiert, die biologisch aktiven Fraktionen konzentriert und an einem Gel mit einem Ausschlussvolumen von unter 3000–4000 Dalton rechromatographiert, durch Eluieren mit Wasser fraktioniert, die aktiven Fraktionen lyophilisiert, das Lyophilisat in einem Puffer des pH-Bereiches von 8,1–8,2 auflöst, zu der Lösung, auf das Gewicht des lyophilisierten Produktes bezogen, 0,01–0,2 Gewichtsteile Trypsin und die gleiche Menge Chymotrypsin gibt, das Gemisch bei 36–39°C 20–30 Stunden lang – vorzugsweise unter gelegentlichem Umrühren – dem enzymatischen Abbau unterzieht, dabei nach Ablauf der ersten 1–10 Stunden dem Gemisch die halbe Menge der zuerst zugesetzten Enzymmengen zugibt und den enzymatischen Abbau fortsetzt, dann dem Reaktionsgemisch bei 0–10°C Trichloressigsäure bis zu einer Konzentration von 4–6 G/V-%, vorzugsweise 5 G/V-%, zusetzt, das Gemisch bei –15°C bis 5°C 1–24 Stunden lang stehen lässt, den unlöslichen Teil abtrennt, das Gemisch an einem Gel mit einem Ausschlussvolumen von unter 3000–4000 Dalton chromatographiert, mit destilliertem Wasser fraktioniert und die biologisch aktiven Fraktionen lyophilisiert.

2. Verfahren zur Herstellung eines spezifisch auf das Appetitzentrum wirkenden, appetitregulierenden Wirkstoffes (Satietin-D) aus menschlichem und/oder tierischem Blutserum, dadurch gekennzeichnet, dass man das menschliche und/ oder tierische Blutserum durch ein bis höchstens zum Molekulargewicht 50 000 durchlässiges Ultrafilter filtriert, das Filtrat partiell eindampft und dann bei 0–10°C bis zum Erreichen einer Konzentration von 5–25 G/V-%, vorzugsweise 9–11 G/V-%, mit Trichloressigsäure versetzt, die ausgefällten Proteine entfernt, die erhaltene Lösung an einem Gel mit einem Ausschlussvolumen von unter 4000 chromatographiert, mit einer Lösung eines pH-Wertes von 6,0–7,0 eluiert, die biologisch aktiven Fraktionen konzentriert und an einem Gel mit einem Ausschlussvolumen von unter 3000–4000 Dalton rechromatographiert, durch Eluieren mit Wasser fraktioniert, die aktiven Fraktionen lyophilisiert, das Lyophilisat in einem Puffer des pH-Bereiches von 8,1–8,2 auflöst, zu der Lösung auf das Gewicht des lyophilisierten Produktes bezogen 0,01–0,2 Gewichtsteile Trypsin und die gleiche Menge Chymotrypsin gibt, das Gemisch bei 36–39°C 20–30 Stunden lang – vorzugsweise unter gelegentlichem Umrühren – dem enzymatischen Abbau unterzieht, dabei nach Ablauf der ersten Stunden dem Gemisch die halbe Menge der zuerst zugesetzten Enzymmengen zugibt und den enzymatischen Abbau fortsetzt, dann dem Reaktionsgemisch bei 0–10°C Trichloressigsäure bis zu einer Konzentration von 4–6 G/V-%, vorzugsweise 5 G/V-%, zusetzt, das Gemisch bei –15°C bis 5°C 1–24 Stunden lang stehen lässt, den unlöslichen Teil abtrennt, das Gemisch an einem Gel mit einem Ausschlussvolumen von unter 3000–4000 Dalton chromatographiert, mit destilliertem Wasser fraktioniert und die biologisch aktiven Fraktionen lyophilisiert.

3. Verfahren nach Anspruch 2, dadurch gekennzeichnet, dass man das menschliche und/ oder tierische Blutserum an einer Säule ultrafiltriert.

4. Verfahren nach Anspruch 2, dadurch gekennzeichnet, dass man in den einzelnen Stufen des Verfahrens die unlöslichen Teile und/oder Eiweisse durch Ultrazentrifugieren oder Vakuumfiltration entfernt.

5. Verfahren nach Anspruch 2, dadurch gekennzeichnet, dass man als Lösung des pH-Bereiches 6,0–7,0 0,1 molare Ammoniumacetatlösung verwendet.

6. Verfahren nach Anspruch 2, dadurch gekennzeichnet, dass man die beim Eluieren mit der Lösung eines pH-Bereiches von 6,0–7,0 erhaltenen aktiven Fraktionen durch Lyophilisieren oder Eindampfen im Vakuum konzentriert.

7. Verfahren nach Anspruch 2, dadurch gekennzeichnet, dass man als Puffer mit einem pH-Wert von 8,1–8,2 0,1 molares 2-Amino-2-hydroxymethyl-1,3-propandiolhydrochlorid verwendet.

8. Verfahren nach Anspruch 2, dadurch gekennzeichnet, dass man zum enzymatischen Abbau der Lösung des lyophilisierten Produktes Trypsin und Chymotrypsin in einer Menge von, bezogen auf das lyophilisierte Produkt, je 0,1 Gew.-Teil zusetzt.

9. Verfahren nach Anspruch 2, dadurch gekennzeichnet, dass man den enzymatischen Abbau bei 37–38°C 24 Stunden lang vornimmt.

**Claims**

1. Appetite-regulating active substance (Satietin-D), which acts specifically upon the appetite centre and is obtained from blood serum, obtainable in that the human and/or animal blood serum is filtered through an ultrafilter permeable up to at most molecular weight 50,000, the filtrate is partially concentrated by evaporation and then mixed at 0–10°C with tri-chloro-acetic acid until a concentration is reached of 5–25% w/v, preferably 9–11% w/v, the precipitated proteins are removed, the obtained solution is chromatographed on a gel with an exclusion volume of below 4,000, eluted with a solution of a pH value of 6.0–7.0, the biologically active fractions are concentrated and rechromatographed on a gel with an exclusion volume of below 3,000–4,000 Dalton, fractionated by elution with water, the active fractions are lyophilised, the lyophilate is dissolved in a buffer of the pH range of 8.1–8.2, 0.01–0.2 parts by weight of trypsin and the same quantity of chymotrypsin, in relation to the weight of the lyophilised product, are added to the solution, the mixture is subjected at 36–39°C for 20–30 hours – preferably with occasional stirring – to enzymatic dissociation, in this action after the elapse of the first 1–10 hours half the quantity of the first-added enzyme quantities is added to the mixture and the enzymatic dissociation is continued, then tri-chloro-acetic acid is added to the reaction mixture at 0–10°C to a concentration of 4–6% w/v, preferably 5% w/v, the mixture is left to stand at –15°C to +5°C for 1–24 hours, the insoluble part is separated, the mixture is chromatographed on a gel with an exclusion volume of below 3,000–4,000 Dalton, fractionated with distilled water and the biologically active fractions are lyophilised.

2. Proces for the production of an appetite-regulating active substance (Satietin-D) acting specifically upon the appetite centre from human and/or animal blood serum, characterised in that the human and/or animal blood serum is filtered through an ultra-filter permeable up to at most molecular weight 50,000, the filtrate is partially concentrated by evaporation and then mixed with tri-chloroacetic acid at 0–10°C until a concentration of 5–25% w/v, preferably 9–11% w/v is reached, the precipitated proteins are removed, the obtained solution is chromatographed on a gel with an exclusion volume of below 4,000, and eluted with a solution of a pH value of 6.0–7.0, the biologically active fractions are concentrated and rechromatographed on a gel with an exclusion volume of below 3,000–4,000 Dalton, fractionated by elution with water, the active fractions are lyophilised, the lyophilate is dissolved in a buffer of the pH range of 8.1–8.2, 0.01–0.2 parts by weight of trypsin, in relation to the weight of the lyophilised product, and the same quantity of chymotrypsin are added to the solution, the mixture is subjected to enzymatic dissociation at 36–39°C for 20–30 hours – preferably with occasional stirring – in this action after the elapse of the first hours half the quantity of the first-added enzyme quantities is added to the mixture and the enzymatic dissociation is continued, then tri-chloro-acetic acid is added to the reaction mixture at 0–10°C up to a concentration of 4–6% w/v, preferably 5% w/v, the mixture is left to stand at –15 – +5°C for 1–24 hours, the insoluble part is separated, the mixture is chromatographed on a gel with an exclusion volume of below 3,000–4,000 Dalton, fractionated with distilled water and the biologically active fractions are lyophilised.

3. Process according to Claim 2, characterised in that the human and/or animal blood serum is ultra-filtered on a column.

4. Process according to Claim 2, characterised in that in the individual stages of the process the insoluble parts and/or proteins are removed by ultra-centrifuging or vacuum filtration.

5. Process according to Claim 2, characterised in that 0.1-molar ammonium acetate solution is used as solution of the pH range 6.0–7.0.

6. Process according to Claim 2, characterised in that the active fractions obtained in elution with the solution of a pH range of 6.0–7.0 are concentrated by lyophilisation or evaporation in vacuo.

7. Process according to Claim 2, characterised in that 0.1-molar 2-amino-2-hydroxymethyl-1.3-propanediolhydrochloride is used as buffer with a pH value of 8.1–8.2.

8. Process according to Claim 2, characterised in that trypsin and chymo-trypsin each in a quantity of 0.1 part by weight, in relation to the lyophilised product, are added to the solution of the lyophilised product for the enzymatic dissociation.

9. Process according to Claim 2, characterised in that the enzymatic dissociation is effected at 37–38°C for 24 hours.

**Revendications**

1. Agent pour le contrôle de l'appétit (Satiétine D) qui agit spécifiquement sur le centre de l'appétit et est obtenu à partir de sérum sanguin, qu'il est possible de préparer de la façon suivante: le sérum sanguin d'origine humaine et/ou animale est filtré sur un ultrafiltre perméable jusqu'à un poids moléculaire de 50 000 au maximum, le filtrat est évaporé partiellement et additionné ensuite à 0–10°C d'acide trichloracétique jusqu'à ce que soit atteinte une concentration de 5–25%, p/v, de préférence 9–11%, p/v, les protéines précipitées sont éliminées, la solution obtenue est chromatographiée sur un gel avec un volume d'élution inférieur à 4000, éluée avec une solution de pH 6,0–7,0, les fractions biologiquement actives sont concentrées et rechromatographiées sur un gel avec un volume d'élution inférieur à 3000–4000 daltons, fractionnées par élution avec de l'eau, les fractions actives sont lyophilisées, le lyophilisat est dissous dans un tampon dans l'intervalle de pH de 8,1–8,2, à la solution, en se basant sur le poids du produit lyophilisé, on ajoute 0,01–0,2 parties en poids de trypsine et la même quantité de chymotrypsine, le mélange est soumis à la dégradation enzymatique à 36–39°C pendant 20–30 heures – de préférence sous agitation intermittente, en ajoutant après les 1–10 premières heures la moitié des quantités d'enzymes ajoutées initialement et la dégradation enzymatique se poursuit, ensuite le mélange réactionnel est additionné à 0–10°C d'acide trichloracétique jusqu'à une concentration de 4–6%, p/v, de préférence 5%, p/v, le mélange est laissé au repos à des températures comprises entre –15°C et 5°C pendant 1 à 24 heures, la partie insoluble est séparée, le mélange est chromatographié sur un gel avec un volume d'élution inférieur à 3000–4000 daltons, fractionné avec de l'eau distillée et les fractions biologiquement actives sont lyophilisées.

2. Procédé de préparation d'un agent pour le contrôle de l'appétit (Satiétine D) agissant spécifiquement sur le centre de l'appétit, obtenu à partir de sérum sanguin d'origine humaine et/ou animale, caractérisé en ce que le sérum sanguin d'origine humaine et/ou animale est filtré sur un ultrafiltre perméable jusqu'à un poids moléculaire de 50 000 au maximum, le filtrat est évaporé partiellement et additionné ensuite à 0–10°C d'acide trichloracétique jusqu'à ce que soit atteinte une concentration de 5–25%, p/v, de préférence 9–11%, p/v, les protéines précipitées sont éliminées, la solution obtenue est chromatographiée sur un gel avec un volume d'élution inférieur à 4000, éluée avec une solution de pH 6,0–7,0, les fractions biologiquement actives sont concentrées et rechromatographiées sur un gel avec un volume d'élution inférieur à 3000–4000 daltons, fractionnées par élution avec de l'eau, les fractions actives sont lyophilisées, le lyophilisat est dissous dans un tampon dans l'intervalle de pH 8,1–8,2, à la solution, en se basant sur le poids du produit lyophilisé, on ajoute 0,01–0,2 parties en poids de trypsine et la même quantité de chymotrypsine, le mélange est soumis à la dégradation enzymatique à 36–39°C pendant 20–30 heures – de préférence sous agitation intermittente, en ajoutant au mélange après les premières heures la moitié des quantités d'enzymes ajoutées initialement et la dégradation enzymatique se poursuit, ensuite le mélange est additionné à 0–10°C d'acide trichloracétique jusqu'à une concentration de 4–6%, p/v, de préférence 5%, p/v, le mélange est laissé au repos à des températures comprises entre –15°C et 5°C pendant 1 à 24 heures, la partie insoluble est séparée, le mélange est chromatographié sur un gel avec un volume d'élution inférieur à 3000–4000 dalton, fractionné avec de l'eau distillée et les fractions actives sont lyophilisées.

3. Procédé selon la revendication 2, caractérisé en ce que le sérum sanguin d'origine humaine et/ou animale est ultrafiltré sur une colonne.

4. Procédé selon la revendication 2, caractérisé en ce que dans les différentes étapes du procédé, les parties et/ou les protéines insolubles sont éliminées par ultracentrifugation ou par filtration sous vide.

5. Procédé selon la revendication 2, caractérisé en ce que l'on utilise comme solution dans l'intervalle de pH 6,0–7,0 une solution d'acétate d'ammonium 0,1 molaire.

6. Procédé selon la revendication 2, caractérisé en ce que les fractions actives obtenues lors de l'élution avec la solution dans l'intervalle de pH 6,0–7,0 sont concentrées par lyophilisation ou évaporation sous vide.

7. Procédé selon la revendication 2, caractérisé en ce que l'on utilise comme tampon avec un pH de 8,1–8,2 du chlorhydrate de 2-amino-2-hydroxyméthyl-1,3-propanediol 0,1 molaire.

8. Procédé selon la revendication 2, caractérisé en ce que pour la dégradation enzymatique, on ajoute à la solution du produit lyophilisé de la trypsine et de la chymotrypsine en quantité, rapportée au produit lyophilisé, de 0,1 partie en poids de chacune.

9. Procédé selon la revendication 2, caractérisé en ce que la dégradation enzymatique est réalisée à 37–38°C pendant 24 heures.

# ISOLATION VON SATIETIN-D

## ULTRAFILTRATION

an Membran Amicon YM-10 oder mittels
High Performance Hollow Fiber System

↓

Ausfällen der Eiweiße mit Trichloressigsäure

↓

Gelchromatographie an Sephadex G-15 und
Bio- Gel P-2

↓

Proteolytischer Abban mit Trypsin und Kimotrypsin

↓

Gelchromatografie an Bio-Gel P-2

↓

Homogenes Produkt

Fig.1

Fig. 2